# EUROPEAN PATENT APPLICATION

(11) **EP 4 715 836 A1**
(43) Date of publication of application: **25.03.2026**
(21) Application number: 24826279.2
(22) Date of filing: 20.06.2024
(51) Int. Cl.: G16H 20/60, G16H 10/60, A61B 5/145, A61B 5/00

(54) **MEAL MONITORING DEVICE AND METHOD THEREOF**

(30) Priority: 20.06.2023 KR 20230078880
(71) Applicant: i-Sens, Inc., Seoul 06646 (KR)
(72) Inventor: LEE, Jae Seon, Seoul 01882 (KR); LEE, Jae Sook, Yongin-si, Gyeonggi-do 16810 (KR)
(74) Representative: Isarpatent
(86) International application number: PCT/KR2024/008553
(87) International publication number: WO 2024/262964

(57) **Abstract**

A meal monitoring apparatus according to an embodiment of the present invention includes a data receiving unit configured to receive measurement data of parameters related to an ear of a target object and blood glucose data of the target object; a first estimated time calculating unit configured to calculate a first estimated time estimated as a food intake activity time of the target object based on the measurement data; a second estimated time calculating unit configured to calculate a second estimated time estimated as a glucose absorption time of the target object based on the blood glucose data; and a meal time calculating unit configured to calculate a meal time of the target object based on the first estimated time and the second estimated time.

## Description

### BACKGROUND OF THE INVENTION

### Field of the Invention

The embodiment relates to an apparatus for monitoring meals and a method thereof.

### Description of the Related Art

Diabetes mellitus is a chronic disease that occurs frequently in modern people, and in the case of the domestic population, it reaches more than two million people, corresponding to five percent of the total population. Diabetes mellitus occurs when insulin, which is produced in the pancreas, becomes absolutely insufficient or relatively insufficient due to various causes such as obesity, stress, improper eating habits, and congenital heredity, thereby failing to properly regulate the balance of sugar in blood, and thus the amount of sugar components in the blood becomes absolutely excessive. Glucose of a certain concentration is normally contained in blood, and tissue cells obtain energy therefrom.

However, when glucose increases beyond what is necessary, it cannot be properly stored in the liver, muscles, fat cells, or the like, and is accumulated in the blood, whereby a diabetic patient maintains a much higher blood glucose level than a normal person, and as the excessive blood glucose passes directly through tissues and is excreted into urine, sugars absolutely required for each tissue of the human body become insufficient, thereby causing abnormalities in respective tissues of the human body.

Diabetes mellitus is characterized by having almost no subjective symptoms in an initial stage, but as the disease progresses, characteristic symptoms specific to diabetes mellitus such as polydipsia, polyphagia, polyuria, weight loss, general fatigue, skin pruritus, and delayed healing of wounds on hands and feet appear, and when the disease further progresses, complications develop, advancing to visual disturbance, hypertension, nephropathy, stroke, periodontal disease, muscle cramp, neuralgia, gangrene, and the like.

In order to diagnose such diabetes mellitus and to prevent it from progressing into complications, not only should systematic blood glucose measurement and treatment be accompanied, but also constant management of blood glucose levels is required. In particular, for accurate prediction and management of blood glucose, information about meals, for example, information about types of food consumed, amounts of food, times of intake, and the like, is essential. For such meal monitoring, in existing systems related to meal management, a method in which a user directly inputs meal time or meal amount has been adopted.

However, a meal monitoring method in which a user directly inputs information regarding a meal is likely to cause omission or erroneous input of meal-related information. Such input omission and erroneous input ultimately act as factors that hinder accurate prediction of changes in blood glucose, thereby causing difficulties in diabetes management.

As background art of the present invention, there exist Japanese Laid-Open Patent Publication No. 2011-062335 and U.S. Patent Application Publication No. US2010/0168537.

### SUMMARY OF THE INVENTION

An embodiment relates to an apparatus for monitoring meals and a method thereof capable of recording and providing meal-related information such as meal time and meal amount with high accuracy.

The objects to be achieved by the embodiments are not limited to the above-mentioned objects, but also include objects or effects that may be understood from the solutions or embodiments described below.

There is provided a meal monitoring apparatus, according to an embodiment of the present invention. The meal monitoring apparatus may comprise: a data receiving unit configured to receive measurement data of parameters related to an ear of a target object and blood glucose data of the target object; a first estimated time calculating unit configured to calculate a first estimated time estimated as a food intake activity time of the target object based on the measurement data; a second estimated time calculating unit configured to calculate a second estimated time estimated as a glucose absorption time of the target object based on the blood glucose data; and a meal time calculating unit configured to calculate a meal time of the target object based on the first estimated time and the second estimated time.

The first estimated time calculating unit may compare a feature value calculated from the measurement data with a first reference value corresponding to the feature value, and calculate the first estimated time based on a comparison result between the feature value and the first reference value.

The second estimated time calculating unit may compare a blood glucose value included in the blood glucose data with a second reference value corresponding to the blood glucose value, and calculate the second estimated time based on a comparison result between the blood glucose value and the second reference value.

The meal time calculating unit may calculate a reference time range based on the second estimated time, select a third estimated time existing within the reference time range among the first estimated times, detect a start estimated time being an earliest time and an end estimated time being a latest time among the third estimated times, and calculate the meal time based on the start estimated time and the end estimated time.

The meal monitoring apparatus may further comprise: a meal amount calculating unit configured to calculate a meal amount ingested by the target object at the meal time based on the measurement data and the blood glucose data.

The measurement data of parameters related to the ear may comprise at least one of vibration signal data, optical signal data, acoustic signal data, and pressure signal data.

The first estimated time calculating unit may generate a harmonic pattern of the vibration signal data based on frequency information included in the vibration signal data, determine whether similarity between the harmonic pattern and a preset reference harmonic pattern is greater than or equal to a first threshold value, determine whether the frequency information is included in a predetermined frequency range, and calculate a time interval in which the similarity is greater than or equal to a predetermined threshold value and the frequency information is included in the predetermined range as the first estimated time.

The first estimated time calculating unit may calculate a amount of change in light by using information about an amount of light included in the optical signal data, compare the amount of change in light with a first threshold range, and calculate a time interval in which the amount of change in light is included in the first threshold range as the first estimated time.

The first estimated time calculating unit may generate an acoustic pattern of the acoustic signal data based on acoustic information included in the acoustic signal data, calculate similarity by comparing the acoustic pattern with a preset reference acoustic pattern, and calculate a time interval in which the similarity is greater than or equal to a second threshold value as the first estimated time.

The first estimated time calculating unit may calculate force and a amount of change in force by using pressure information included in the pressure signal data, determine whether a magnitude of force is greater than or equal to a third threshold value, determine whether the amount of change in force is included in a second threshold range, and calculate a time interval in which the magnitude of force is greater than or equal to the third threshold value and the amount of change in force is included in the second threshold range as the first estimated time.

There is provided a meal monitoring method, according to an embodiment of the present invention. The meal monitoring method may comprise: receiving measurement data of parameters related to an ear of a target object and blood glucose data of the target object; calculating a first estimated time estimated as a food intake activity time of the target object based on the measurement data; calculating a second estimated time estimated as a glucose absorption time of the target object based on the blood glucose data; and calculating a meal time of the target object based on the first estimated time and the second estimated time.

The calculating of the first estimated time may comprise: comparing a feature value calculated from the measurement data with a first reference value corresponding to the feature value; and calculating the first estimated time based on a comparison result between the feature value and the first reference value.

The calculating of the second estimated time may comprise: comparing a blood glucose value included in the blood glucose data with a second reference value corresponding to the blood glucose value; and calculating the second estimated time based on a comparison result between the blood glucose value and the second reference value.

The calculating of the meal time of the target object may comprise: calculating a reference time range based on the second estimated time; selecting a third estimated time existing within the reference time range among the first estimated times; detecting a start estimated time being an earliest time and an end estimated time being a latest time among the third estimated times; and calculating the meal time based on the start estimated time and the end estimated time.

The meal monitoring method may further comprise: calculating a meal amount ingested by the target object at the meal time based on the measurement data and the blood glucose data.

The measurement data of parameters related to the ear may comprise at least one of vibration signal data, optical signal data, acoustic signal data, and pressure signal data.

The calculating of the first estimated time may comprise: generating a harmonic pattern of the vibration signal data based on frequency information included in the vibration signal data; determining whether similarity between the harmonic pattern and a preset reference harmonic pattern is greater than or equal to a first threshold value; determining whether the frequency information is included in a predetermined frequency range; and calculating a time interval in which the similarity is greater than or equal to a predetermined threshold value and the frequency information is included in the predetermined range as the first estimated time.

The calculating of the first estimated time may comprise: calculating a amount of change in light by using information about an amount of light included in the optical signal data; comparing the amount of change in light with a first threshold range; and calculating a time interval in which the amount of change in light is included in the first threshold range as the first estimated time.

The calculating of the first estimated time may comprise: generating an acoustic pattern of the acoustic signal data based on acoustic information included in the acoustic signal data; calculating similarity by comparing the acoustic pattern with a preset reference acoustic pattern; and calculating a time interval in which the similarity is greater than or equal to a second threshold value as the first estimated time.

The calculating of the first estimated time may comprise: calculating force and a amount of change in force by using pressure information included in the pressure signal data; determining whether a magnitude of force is greater than or equal to a third threshold value; determining whether the amount of change in force is included in a second threshold range; and calculating a time interval in which the magnitude of force is greater than or equal to the third threshold value and the amount of change in force is included in the second threshold range as the first estimated time.

According to an embodiment, accurate information related to meals, such as meal time and meal amount, is provided to a user.

In addition, through the provision of accurate information regarding meal time and meal amount, a change in blood glucose may be predicted more accurately.

Further, since information such as meal time and meal amount is recorded and provided without intervention of the user, convenience in meal monitoring is provided to the user.

The various, beneficial advantages and effects of the disclosure are not limited to the above-mentioned contents and may be more easily understood during the process of describing the specific embodiments of the disclosure.

### BRIEF DESCRIPTION OF THE DRAWINGS

FIG. 1 is a configuration diagram illustrating an embodiment of a meal monitoring system according to an embodiment of the present invention.
FIG. 2 is a first embodiment of a sensing apparatus according to an embodiment of the present invention.
FIG. 3 is a second embodiment of a sensing apparatus according to an embodiment of the present invention.
FIG. 4 is a third embodiment of a sensing apparatus according to an embodiment of the present invention.
FIG. 5 is a fourth embodiment of a sensing apparatus according to an embodiment of the present invention.
FIG. 6 is a configuration diagram of a meal monitoring apparatus according to an embodiment of the present invention.
FIG. 7 is a flowchart of a meal monitoring method according to an embodiment of the present invention.
FIG. 8 is a flowchart illustrating in detail step S720 of FIG. 7.
FIG. 9 is a flowchart illustrating a first embodiment of step S720 of FIG. 7.
FIG. 10 is a flowchart illustrating a second embodiment of step S720 of FIG. 7.
FIG. 11 is a flowchart illustrating a third embodiment of step S720 of FIG. 7.
FIG. 12 is a flowchart illustrating a fourth embodiment of step S720 of FIG. 7.
FIG. 13 is a diagram for explaining a process of calculating meal time according to an embodiment of the present invention.

### DETAILED DESCRIPTION OF THE INVENTION

The present invention may be variously modified and may have various embodiments, and particular embodiments illustrated in the drawings will be described below. However, the description of the embodiments is not intended to limit the disclosure to the particular embodiments, but it should be understood that the disclosure is to cover all modifications, equivalents and alternatives falling within the technical teachings and technical scope of the disclosure.

The terms including ordinal numbers such as 'second', 'first', and the like may be used to describe various constituent elements, but the constituent elements are not limited by the terms. These terms are used only to distinguish one constituent element from another constituent element. For example, a second component may be named a first component, and similarly, the first component may also be named the second component, without departing from the scope of the disclosure. The term "and/or" includes any and all combinations of a plurality of the related and listed items.

When one constituent element is described as being "coupled" or "connected" to another constituent element, it should be understood that one constituent element can be coupled or connected directly to another constituent element, and an intervening constituent element can also be present between the constituent elements. When one constituent element is described as being "coupled directly to" or "connected directly to" another constituent element, it should be understood that no intervening constituent element exists between the constituent elements.

The terminology used herein is used for the purpose of describing particular embodiments only and is not intended to limit the present invention. Singular expressions include plural expressions unless clearly described as different meanings in the context. The terms "comprises," "comprising," "includes," "including," "containing," "has," "having" or other variations thereof are inclusive and therefore specify the presence of stated features, integers, steps, operations, constituent elements, and/or components, but do not preclude the presence or addition of one or more other features, integers, steps, operations, constituent elements, components, and/or groups thereof.

Unless otherwise defined, all terms used herein, including technical or scientific terms, have the same meaning as commonly understood by those skilled in the art to which the present invention pertains. The terms such as those defined in a commonly used dictionary should be interpreted as having meanings consistent with meanings in the context of related technologies and should not be interpreted as ideal or excessively formal meanings unless explicitly defined in the present application.

Hereinafter, embodiments will be described in detail with reference to the accompanying drawings. The same or corresponding constituent elements are assigned with the same reference numerals throughout the drawings, and the repetitive description thereof will be omitted.

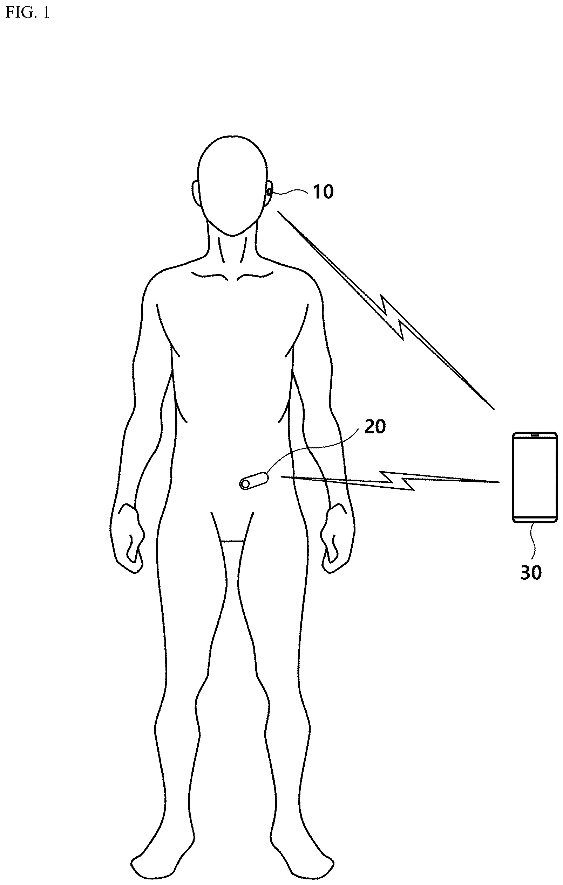

FIG. 1 is a configuration diagram illustrating an embodiment of a meal monitoring system according to an embodiment of the present invention.

A meal monitoring system according to an embodiment of the present invention calculates a meal time of a target object and provides the calculated meal time to a user. Here, the meal monitoring system may use measurement data of parameters related to an ear of the target object and blood glucose data of the target object in calculating the meal time of the target object.

When the meal monitoring system according to an embodiment of the present invention calculates and provides the meal time of the target object, the user may utilize the calculated meal time as information necessary for managing the health of the target object. For example, the calculated meal time may be used to determine whether a rise in blood glucose of the user occurred due to food intake or occurred due to a cause other than a meal, such as infection. As another example, through a meal amount that may be provided together with the meal time, it may also be used to determine whether an appropriate amount of food for blood glucose management has been consumed. Here, the user means an entity that receives information about the calculated meal time of the target object. The user may be a person who is the target object. In addition, the user may be a medical professional, such as a doctor or nurse, who manages the health of the target object (healthcare personnel, HCP). In addition, the user may be a family member or guardian of the target object. In addition, the user may also be a server system that performs a function of managing the health of the target object or collecting and analyzing health management information and the like.

With reference to FIG. 1, the meal monitoring system according to an embodiment of the present invention includes a sensing apparatus 10, a blood glucose measuring apparatus 20, and a meal monitoring apparatus 30.

First, the sensing apparatus 10 may mean an apparatus that measures parameters related to an ear of the target object and generates measurement data. Here, the target object may mean a person, but is not limited thereto. The target object may include an animal other than a person. Further, the parameters related to an ear may mean parameters that occur in the ear as the target object ingests food. According to one embodiment, parameters related to an ear may include vibration signals generated from an earcanal, an eardrum, and the like as a target object ingests food. According to one embodiment, parameters related to an ear may include optical signals reflected from an earcanal, an eardrum, and the like as a target object ingests food. According to one embodiment, parameters related to an ear may include acoustic signals generated from an earcanal, an eardrum, and the like as a target object ingests food. According to one embodiment, parameters related to an ear may include pressure signals generated from an earcanal, an eardrum, and the like as a target object ingests food.

The sensing apparatus 10 may be disposed on an ear of a target object to measure parameters related to the ear. According to one embodiment, the sensing apparatus 10 may be implemented in a form of a device such as an earphone or headphone. The sensing apparatus 10 may also be implemented in a form combined with a device such as an earphone or headphone.

The sensing apparatus 10 may be communicatively connected with a meal monitoring apparatus 30. The sensing apparatus 10 may be communicatively connected with the meal monitoring apparatus 30 by wireless and/or wired communication. The sensing apparatus 10 may include wireless and/or wired communication apparatuses for being communicatively connected with the meal monitoring apparatus 30. For example, the sensing apparatus 10 may include a USB cable device, an infrared communication device, a Near Field Communication (NFC) device, a Bluetooth device, a Wireless Fidelity (WiFi) device, an LTE device, and the like.

Next, the blood glucose measuring apparatus 20 may mean an apparatus that measures a glucose level of a target object and generates blood glucose data. Accordingly, the blood glucose data may include information related to the glucose level of the target object. The blood glucose data may be time-series data generated by continuously measuring the glucose level of the target object. Here, continuously means not only measuring the glucose level linearly but also measuring the glucose level at predetermined time intervals. For example, measuring a glucose level sixty times during ten minutes, that is, measuring every ten seconds, may be interpreted as continuously measuring the glucose level.

The blood glucose measuring apparatus 20 may be a continuous blood glucose measuring apparatus (Continuous Glucose Monitoring System, CGMS). According to one embodiment, the blood glucose measuring apparatus 20 may be an apparatus that measures blood glucose of a target object through interstitial fluid existing under the skin. In another embodiment, the blood glucose measuring apparatus 20 may be an apparatus that continuously measures blood glucose of a target object from body fluids such as saliva or tears. In another embodiment, the blood glucose measuring apparatus 20 may be an apparatus that continuously measures blood glucose of a target object from blood. In addition to the above, various apparatuses capable of continuously measuring blood glucose of a target object may be included as examples of the blood glucose measuring apparatus 20.

The blood glucose measuring apparatus 20 may include various apparatus configurations for measuring blood glucose of a target object. According to one embodiment, the blood glucose measuring apparatus 20 may include a transmitter including a sensor that measures a glucose measurement signal of a target object, and a receiver that receives the measured glucose signal and analyzes a blood glucose level. The transmitter and the receiver may be implemented as separate apparatuses, but are not limited thereto, and may also be implemented together in a single apparatus. In addition, the receiver of the blood glucose measuring apparatus 20 may be implemented as the same apparatus as the meal monitoring apparatus 30. In addition to the above, the blood glucose measuring apparatus 20 may be implemented in various types of apparatus configurations.

The blood glucose measuring apparatus 20 may be communicatively connected with the meal monitoring apparatus 30. The blood glucose measuring apparatus 20 may be communicatively connected with the meal monitoring apparatus 30 by wireless and/or wired communication. The blood glucose measuring apparatus 20 may include a communication apparatus according to a communication method for being communicatively connected with the meal monitoring apparatus 30. For example, the blood glucose measuring apparatus 20 may include a USB cable device, an infrared communication device, a Near Field Communication (NFC) device, a Bluetooth device, a Wireless Fidelity (WiFi) device, an LTE device, and the like.

Next, the meal monitoring apparatus 30 may be an apparatus that calculates a meal time of a target object. Here, the meal time means a time during which a target object performs a meal, that is, a time of ingesting food. An act of ingesting food includes all acts in which food is delivered from a mouth to an esophagus, such as chewing food and swallowing food.

The meal monitoring apparatus 30 may calculate a meal time of a target object by using measurement data of parameters related to an ear of the target object and blood glucose data of the target object. For this purpose, the meal monitoring apparatus 30 may receive measurement data and blood glucose data from the sensing apparatus 10 and the blood glucose measuring apparatus 20.

The meal monitoring apparatus 30 may include a communication apparatus for being communicatively connected with the sensing apparatus 10 and the blood glucose measuring apparatus 20 by wireless and/or wired communication. For example, the meal monitoring apparatus 30 may include a USB cable device, an infrared communication device, a Near Field Communication (NFC) device, a Bluetooth device, a Wireless Fidelity (WiFi) device, an LTE device, and the like.

Further, the meal monitoring apparatus 30 may include a processor and a memory for processing measurement data and blood glucose data. Accordingly, the meal monitoring apparatus 30 may be implemented as various types of computing apparatuses including the processor and the memory.

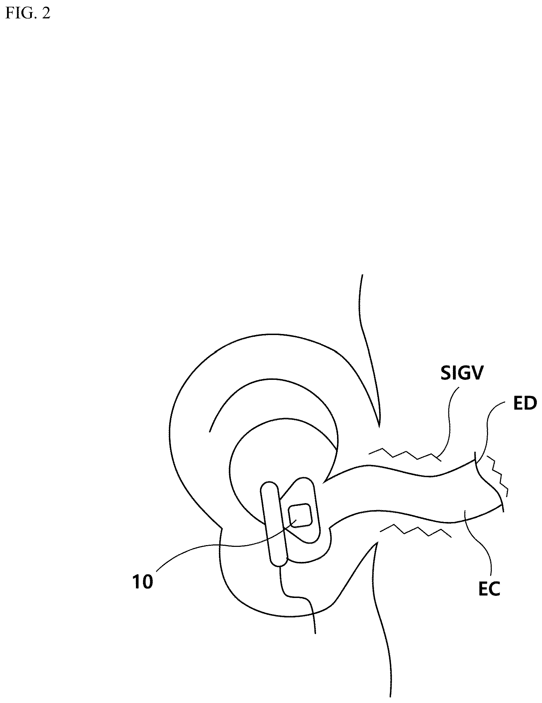

FIG. 2 is a first embodiment of a sensing apparatus according to an embodiment of the present invention.

In FIG. 2, a target object is assumed to be a person for explanation. The sensing apparatus 10 illustrated in FIG. 2 may be an apparatus that measures vibration signal data among parameters related to an ear of a person.

A person's meal act may be classified into mastication and swallowing. In a case of mastication, contact or friction between lower teeth and upper teeth occurs during a process of chewing food. In this case, vibration (SIGV) occurs on an outer wall of an eardrum (ED) and an earcanal (EC) of an ear due to contact and friction between teeth. In a case of swallowing, food moves to a stomach through a mouth, a pharynx, and an esophagus. In this case, during a process in which food moves in order of a mouth, a pharynx, and an esophagus, vibration (SIGV) occurs on an outer wall of an eardrum (ED) and an earcanal (EC) of an ear connected to the mouth, the pharynx, the esophagus, and the like.

The sensing apparatus 10 illustrated in FIG. 2 measures vibration (SIGV) of an ear generated by a meal act. The sensing apparatus 10 may be disposed on an ear of a person and measure vibration (SIGV) generated from an outer wall of an earcanal (EC) and an eardrum (ED) occurring during a meal, thereby generating vibration (SIGV) signal data. The sensing apparatus 10 may directly measure vibration (SIGV) from a skin surface of an ear, but is not limited thereto. The sensing apparatus 10 may also generate a vibration (SIGV) signal by measuring vibration (SIGV) of an ear generated by a meal act through vibration (SIGV) of air in an earcanal (EC).

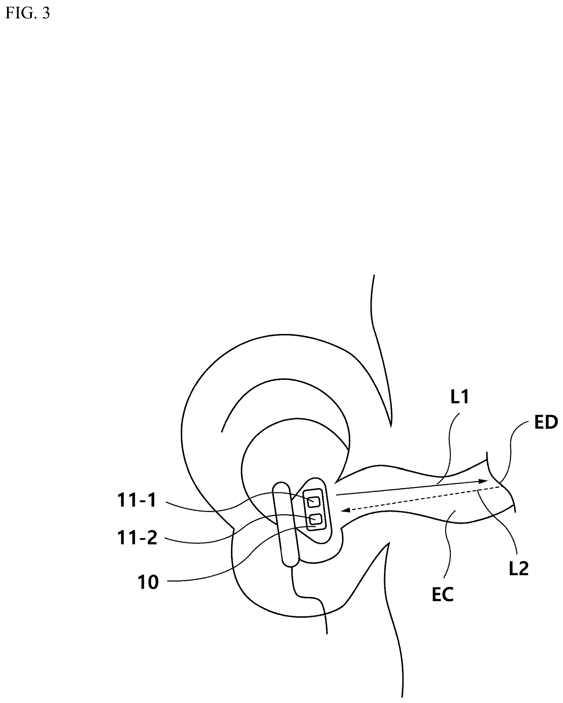

FIG. 3 is a second embodiment of a sensing apparatus according to an embodiment of the present invention.

In FIG. 3, a target object is assumed to be a person for explanation. The sensing apparatus 10 illustrated in FIG. 3 may be an apparatus that measures optical signal data among parameters related to an ear.

A person's meal act may be classified into mastication and swallowing. In a case of mastication, contact or friction between lower teeth and upper teeth occurs during a process of chewing food. In this case, deformation occurs in a shape of an eardrum (ED) and an earcanal (EC) of an ear due to contact and friction between teeth. In a case of swallowing, food moves to a stomach through a mouth, a pharynx, and an esophagus. In this case, during a process in which food moves in order of a mouth, a pharynx, and an esophagus, deformation occurs in the shape of an eardrum (ED) and an earcanal (EC) of an ear connected to the mouth, the pharynx, the esophagus, and the like.

The sensing apparatus 10 illustrated in FIG. 3 measures shape deformation of an eardrum (ED) and an earcanal (EC) of an ear generated by a meal act and generates data. Specifically, the sensing apparatus 10 may generate data by emitting output light (L1) toward an earcanal (EC) of an ear and receiving reflected light (L2) reflected from an outer wall of the earcanal (EC) and an eardrum (ED) of an ear to measure the shape deformation. That is, the sensing apparatus 10 may generate optical signal data corresponding to shape deformation of an ear.

For generating optical signal data, the sensing apparatus 10 may include a light-emitting unit 11-1 and a light-receiving unit 11-2. The light-emitting unit 11-1 may emit output light (L1) having a predetermined frequency toward an earcanal (EC), and the light-receiving unit 11-2 may receive reflected light (L2) reflected within an ear from the output light (L1) emitted by the light-emitting unit 11-1. The sensing apparatus 10 may generate optical signal data by electrically converting an amount of reflected light (L2) received.

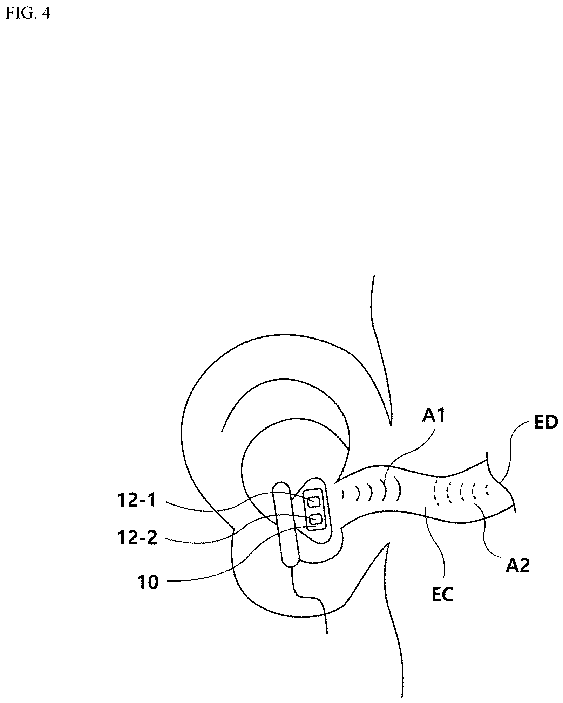

FIG. 4 is a third embodiment of a sensing apparatus according to an embodiment of the present invention.

The sensing apparatus 10 illustrated in FIG. 4 may be an apparatus that measures acoustic signal data among parameters related to an ear.

A person's meal act may be classified into mastication and swallowing. In a case of mastication, contact or friction between lower teeth and upper teeth occurs during a process of chewing food. In this case, deformation occurs in a shape of an eardrum (ED) and an earcanal (EC) of an ear due to contact and friction between teeth. In a case of swallowing, food moves to a stomach through a mouth, a pharynx, and an esophagus. In this case, during a process in which food moves in order of a mouth, a pharynx, and an esophagus, deformation occurs in the shape of an eardrum (ED) and an earcanal (EC) of an ear connected to the mouth, the pharynx, the esophagus, and the like.

The sensing apparatus 10 shown in FIG. 4 measures shape deformation of an eardrum (ED) and an earcanal (EC) of an ear generated by a meal act and generates data. Specifically, the sensing apparatus 10 may generate data by emitting output sound waves (A1) toward an earcanal (EC) of an ear and receiving reflected sound waves (A2) reflected from an outer wall of the earcanal (EC) and an eardrum (ED) of an ear to measure shape deformation. That is, the sensing apparatus 10 may generate acoustic signal data corresponding to shape deformation of an ear.

For generating acoustic signal data, the sensing apparatus 10 may include a speaker 12-1 and a microphone 12-2. The speaker 12-1 means a device for emitting sound. The speaker 12-1 may emit output sound waves (A1) having a predetermined frequency toward an earcanal (EC). According to one embodiment, the output sound waves (A1) emitted from the speaker 12-1 may have a frequency range of 15 kHz or higher. In another embodiment, the output sound waves (A1) emitted from the speaker 12-1 may have a frequency range of 20 kHz or higher. Since acoustic signals in such frequency ranges as the above embodiments are difficult for a person to perceive, disturbance in daily life due to operation of the sensing apparatus 10 may be minimized. The microphone 12-2 means a device for receiving sound. The microphone 12-2 may receive sound waves having a predetermined frequency. The sensing apparatus 10 may generate time-series data of received reflected sound waves (A2). A frequency range of reflected sound waves (A2) received by the microphone 12-2 may differ from a frequency range of output sound waves (A1) emitted by the speaker 12-1.

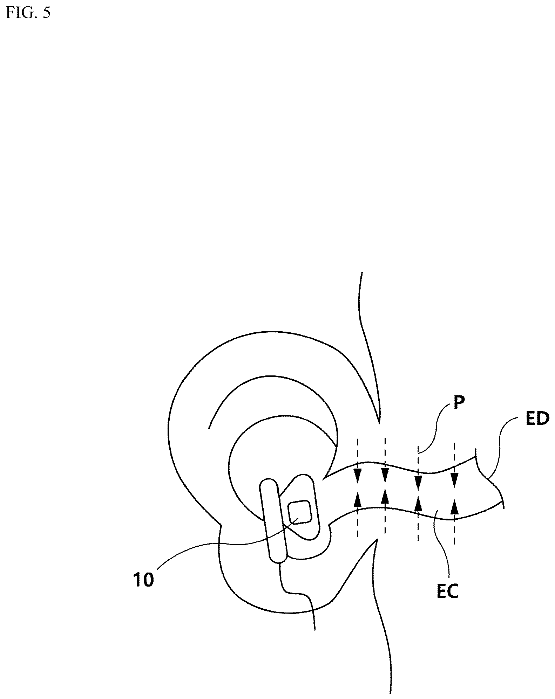

FIG. 5 is a fourth embodiment of a sensing apparatus according to an embodiment of the present invention.

The sensing apparatus 10 illustrated in FIG. 5 may be an apparatus that measures pressure signal data among parameters related to an ear.

A person's meal act may be classified into mastication and swallowing. In a case of mastication, contact or friction between lower teeth and upper teeth occurs during a process of chewing food. In this case, due to contact and friction between teeth, a change in pressure occurs inside an earcanal (EC) of an ear according to a shape change of an earcanal (EC) and an eardrum (ED). In a case of swallowing, food moves to a stomach through a mouth, a pharynx, and an esophagus. In this case, during a process in which food moves in order of a mouth, a pharynx, and an esophagus, the shape of an earcanal (EC) and an eardrum (ED) of an ear connected to the mouth, the pharynx, the esophagus, and the like changes, and accordingly, a change in pressure (P) occurs inside the earcanal (EC).

The sensing apparatus 10 illustrated in FIG. 5 measures pressure (P) of an ear generated by a meal act. The sensing apparatus 10 may be disposed on an ear of a person and may measure pressure (P) inside an earcanal (EC) generated during a meal, thereby generating pressure signal data.

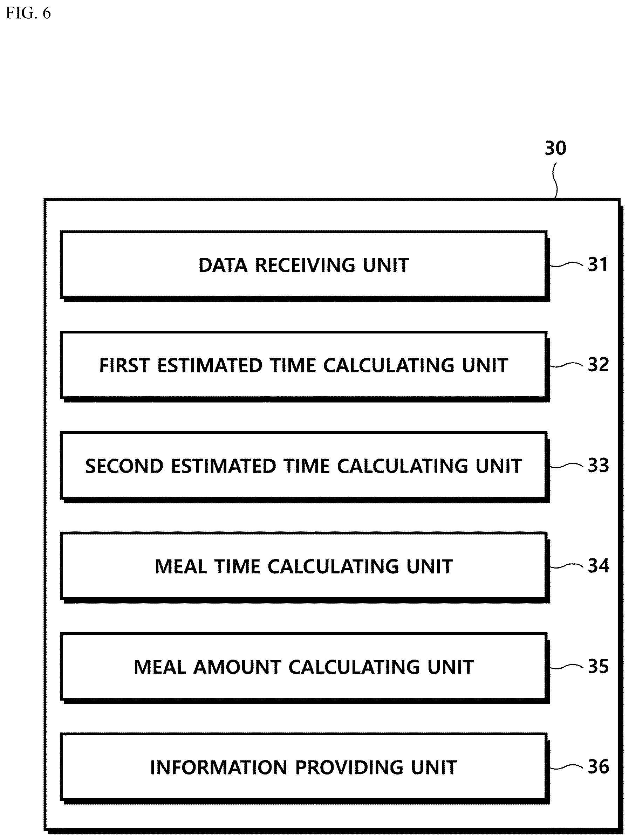

FIG. 6 is a configuration diagram of a meal monitoring apparatus according to an embodiment of the present invention.

With reference to FIG. 6, the meal monitoring apparatus 30 according to an embodiment of the present invention includes a data receiving unit 31, a first estimated time calculating unit 32, a second estimated time calculating unit 33, and a meal time calculating unit 34. In addition, the meal monitoring apparatus 30 may further include a meal amount calculating unit 35 and an information providing unit 36.

First, the data receiving unit 31 receives measurement data of parameters related to an ear of a target object and blood glucose data of the target object. Here, the measurement data of parameters related to the ear may include at least one of vibration signal data, optical signal data, acoustic signal data, and pressure signal data.

Next, the first estimated time calculating unit 32 calculates a first estimated time estimated as a food intake activity time of a target object based on measurement data.

Specifically, the first estimated time calculating unit 32 may compare a feature value calculated from measurement data with a first reference value corresponding to the feature value. The feature value may differ depending on a type of measurement data, and the first reference value may differ depending on each feature value. Further, the first estimated time calculating unit 32 may calculate the first estimated time based on a comparison result between the feature value and the first reference value.

Various types of measurement data generated from an ear may also be measured according to various activities of a target object in addition to a food intake act. For example, measurement data may be generated according to various activities such as conversation, coughing, or walking of a target object. Therefore, in order to improve the accuracy of the first estimated time, accuracy of distinguishing between a meal act and a non-meal act needs to be improved. Accordingly, the present invention improves accuracy of the first estimated time by using various embodiments.

According to one embodiment, measurement data may be vibration signal data. The first estimated time calculating unit 32 may generate a harmonic pattern of the vibration signal data based on frequency information included in the vibration signal data. Further, the first estimated time calculating unit 32 may determine whether similarity between the harmonic pattern and a preset reference harmonic pattern is greater than or equal to a first threshold value. Further, the first estimated time calculating unit 32 may determine whether frequency information is included in a predetermined frequency range. Then, the first estimated time calculating unit 32 may calculate a time interval in which similarity is greater than or equal to a first threshold value and frequency information is included in the predetermined range as a first estimated time.

In general, vibration generated from an ear (eardrum, earcanal, etc.) may also be generated through various acts such as conversation, coughing, or walking other than a food intake act. Accordingly, the present invention distinguishes non-meal acts from meal acts by using harmonic patterns of vibration signals according to meal acts such as chewing and swallowing. However, in view of the diversity of non-meal acts, non-meal acts may have harmonic patterns similar to those of meal acts. Therefore, the present invention improves accuracy of estimating meal act time by comparing frequency ranges in addition to comparison of harmonic patterns.

According to another embodiment, measurement data may be optical signal data. The first estimated time calculating unit 32 may calculate a amount of change in light by using information about an amount of light included in the optical signal data. Further, the first estimated time calculating unit 32 may compare the amount of change in light with a first threshold range. Then, the first estimated time calculating unit 32 may calculate a time interval in which the amount of change in light is included in the first threshold range as a first estimated time.

In general, a shape of an ear (eardrum, earcanal, etc.) may also change through various acts such as conversation, coughing, or walking other than a food intake act. A shape change of an ear causes a change in a distance from an arbitrary point (for example, an entrance of an earcanal) to an eardrum or a distance to an outer wall of an earcanal. Such shape changes of an ear may have similar forms depending on respective acts. Therefore, the present invention distinguishes meal acts from non-meal acts by comparing a change amount of reflected light with a predetermined threshold range, thereby improving accuracy of estimating a meal act time.

According to another embodiment, measurement data may be acoustic signal data. The first estimated time calculating unit 32 may generate an acoustic pattern of acoustic signal data based on acoustic information included in the acoustic signal data. Further, the first estimated time calculating unit 32 may calculate similarity by comparing the acoustic pattern with a preset reference acoustic pattern. Then, the first estimated time calculating unit 32 may calculate a time interval in which similarity is greater than or equal to a second threshold value as a first estimated time.

In general, a shape of an ear (eardrum, earcanal, etc.) may also change through various acts such as conversation, coughing, or walking other than a food intake act. A shape change of an ear causes a change in a distance from an arbitrary point (for example, an entrance of an earcanal) to an eardrum or a distance to an outer wall of an earcanal. Such shape changes of an ear may have similar forms depending on respective acts. Therefore, the present invention distinguishes meal acts from non-meal acts by comparing a pattern of reflected sound waves with a predetermined reference pattern, thereby improving accuracy of estimating a meal act time.

According to another embodiment, measurement data may be pressure signal data. The first estimated time calculating unit 32 may calculate force and a amount of change in force by using pressure information included in pressure signal data. Further, the first estimated time calculating unit 32 may determine whether a magnitude of force is greater than or equal to a third threshold value. Further, the first estimated time calculating unit 32 may determine whether the amount of change in force is included in a second threshold range. The first estimated time calculating unit 32 may calculate a time interval in which the magnitude of force is greater than or equal to the third threshold value and the amount of change in force is included in the second threshold range as a first estimated time.

In general, pressure generated from an ear (eardrum, earcanal, etc.) may also be generated through various acts such as conversation, coughing, or walking other than a food intake act. Accordingly, the present invention distinguishes non-meal acts from meal acts by using an energy amount calculated from pressure signals according to meal acts such as chewing and swallowing. In addition, in view of the diversity of non-meal acts, non-meal acts may have energy amounts similar to those of meal acts. Therefore, the present invention distinguishes meal acts from non-meal acts by comparing a duration of energy amount with a predetermined range value in addition to comparison of energy amounts, thereby improving accuracy of estimating meal act time.

The above-described embodiments related to the first estimated time calculating unit 32 may be implemented independently, but are not limited thereto. The embodiments related to the first estimated time calculating unit 32 may be implemented as a combination of at least two embodiments.

Next, the second estimated time calculating unit 33 calculates a second estimated time estimated as a glucose absorption time of a target object based on blood glucose data.

Specifically, the second estimated time calculating unit 33 may compare a blood glucose value included in blood glucose data with a second reference value corresponding to the blood glucose value. Here, the second reference value may be set based on an expected blood glucose amount that may increase according to food intake, and may be set to be customized to a user. Further, the second estimated time calculating unit 33 may calculate the second estimated time based on a comparison result between the blood glucose value and the second reference value. For example, the second estimated time calculating unit 33 may calculate a time interval in which the blood glucose value is greater than or equal to the second reference value as a second estimated time.

Next, the meal time calculating unit 34 calculates a meal time of a target object based on a first estimated time and a second estimated time.

Specifically, the meal time calculating unit 34 may calculate a reference time range based on the second estimated time. Then, the meal time calculating unit 34 may select a third estimated time existing within the reference time range among the first estimated times. Further, the meal time calculating unit 34 may detect a start estimated time, which is an earliest time, and an end estimated time, which is a latest time, among the third estimated times. Further, the meal time calculating unit 34 may calculate the meal time based on the start estimated time and the end estimated time.

Even when the first estimated time calculating unit 32 calculates an estimated time determined as a food intake activity, due to various activity acts other than the food intake act, a time of non-meal acts may also be calculated as a first estimated time. Accordingly, the present invention improves accuracy of predicting meal time by calculating the meal time of a target object through comparison between the first estimated time and the second estimated time.

The meal amount calculating unit 35 calculates a meal amount ingested by the target object during the meal time based on measurement data and blood glucose data.

Specifically, the meal amount calculating unit 35 may calculate the meal amount ingested by the target object during the meal time calculated based on a blood glucose value included in the blood glucose data. According to one embodiment, the meal amount calculating unit 35 may calculate the meal amount by applying an average value of blood glucose values corresponding to the meal time to an algorithm for calculating meal amount. In this case, the algorithm for calculating meal amount may be pre-stored in the meal monitoring apparatus 30.

The information providing unit 36 provides at least one of calculated meal time information and meal amount information.

According to one embodiment, the information providing unit 36 may provide at least one of the meal time information and meal amount information by using a display device or an acoustic device mounted in the meal monitoring apparatus 30 itself. According to another embodiment, the information providing unit 36 may provide at least one of the meal time information and the meal amount information to an external terminal device communicatively connected with the meal monitoring apparatus 30.

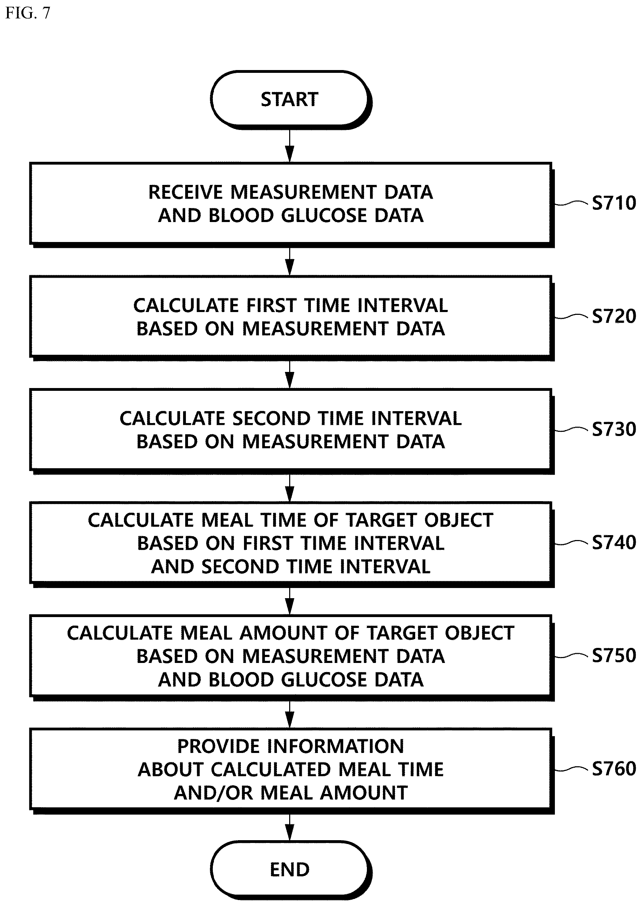

FIG. 7 is a flowchart of a meal monitoring method according to an embodiment of the present invention.

The meal monitoring method of FIG. 7 may be performed by using the meal monitoring apparatus 30 described above.

With reference to FIG. 7, the meal monitoring method according to an embodiment of the present invention includes steps S710 to S740, and may further include steps S750 to S760.

First, the data receiving unit 31 receives measurement data of parameters related to an ear of a target object and blood glucose data of the target object (S710). As described above, the measurement data may include at least one of vibration signal data, optical signal data, acoustic signal data, and pressure signal data.

Next, the first estimated time calculating unit 32 calculates a first estimated time estimated as a food intake activity time of a target object based on measurement data (S720).

Next, the second estimated time calculating unit 33 calculates a second estimated time estimated as a glucose absorption time of the target object based on blood glucose data (S730).

Next, the meal time calculating unit 34 calculates a meal time of the target object based on the first estimated time and the second estimated time (S740).

Next, the meal time calculating unit 34 may calculate a meal amount ingested by the target object during the meal time based on measurement data and blood glucose data (S750).

Next, the information providing unit 36 may provide at least one of calculated meal time information and meal amount information (S760).

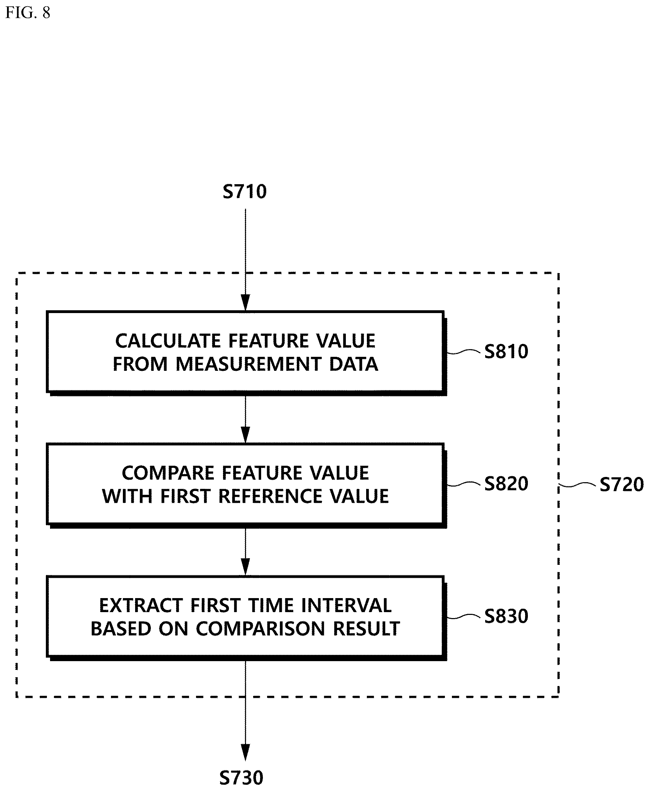

FIG. 8 is a flowchart illustrating step S720 of FIG. 7 in detail.

With reference to FIG. 8, step S720 according to an embodiment of the present invention may include steps S810 to S830.

First, the first estimated time calculating unit 32 may calculate a feature value from measurement data (S810). Here, the feature value may vary depending on a type, kind, or the like of the measurement data.

Next, the first estimated time calculating unit 32 may compare a feature value calculated from the measurement data with a first reference value corresponding to the feature value (S820). Here, the first reference value may be preset to correspond to each feature value.

Next, the first estimated time calculating unit 32 may calculate a first estimated time based on a comparison result between the feature value and the first reference value (S830).

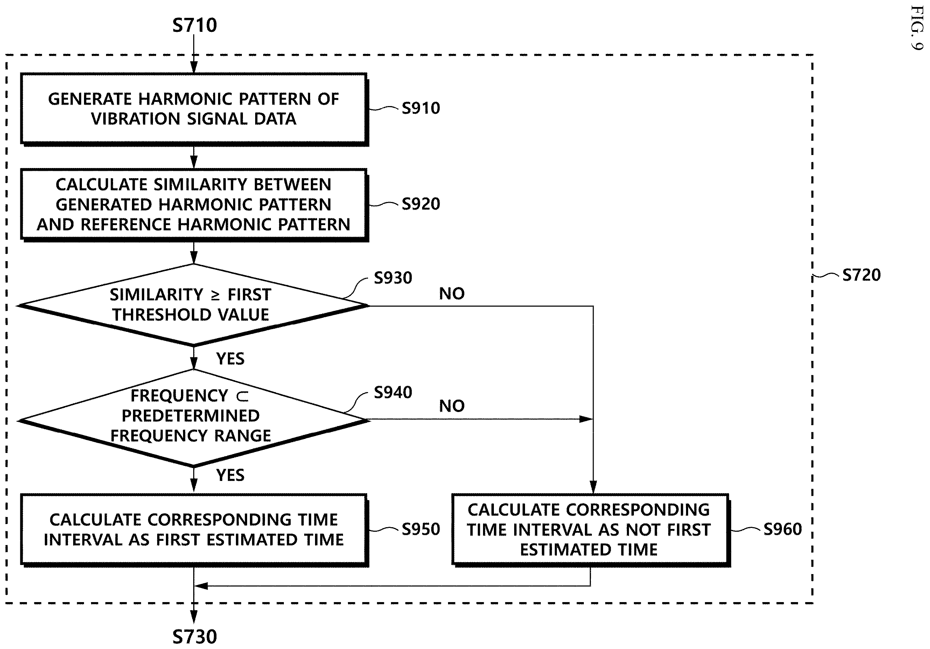

FIG. 9 is a flowchart illustrating a first embodiment of step S720 of FIG. 7.

FIG. 9 is a flowchart for detailed description in a case where the measurement data is vibration signal data. With reference to FIG. 9, step S720 of FIG. 7 may include steps S910 to S960.

First, the first estimated time calculating unit 32 may generate a harmonic pattern of vibration signal data based on frequency information included in the vibration signal data (S910).

Next, the first estimated time calculating unit 32 may calculate a similarity between the harmonic pattern generated from the vibration signal data and a preset reference harmonic pattern (S920).

Next, the first estimated time calculating unit 32 may determine whether the similarity between the harmonic pattern and the preset reference harmonic pattern is greater than or equal to a first threshold value (S930).

When the similarity is greater than or equal to the predetermined threshold value, the first estimated time calculating unit 32 may determine whether frequency information is included in a predetermined frequency range (S940).

When the frequency information is included in the predetermined frequency range, the first estimated time calculating unit 32 may calculate a time interval, in which the similarity is greater than or equal to the first threshold value and the frequency information is included in a predetermined range, as a first estimated time (S950).

On the other hand, if the similarity is less than the first threshold value, or even if the similarity is greater than or equal to the first threshold value, but when the frequency information is not included in the predetermined frequency range, the first estimated time calculating unit 32 may calculate the corresponding time interval as not being a first estimated time (S960).

For convenience of description, the S940 step is described as being performed after the S930 step, but is not limited thereto. As another example, the S940 step may be performed before the S930 step, or the S930 step and the S940 step may be substantially performed simultaneously.

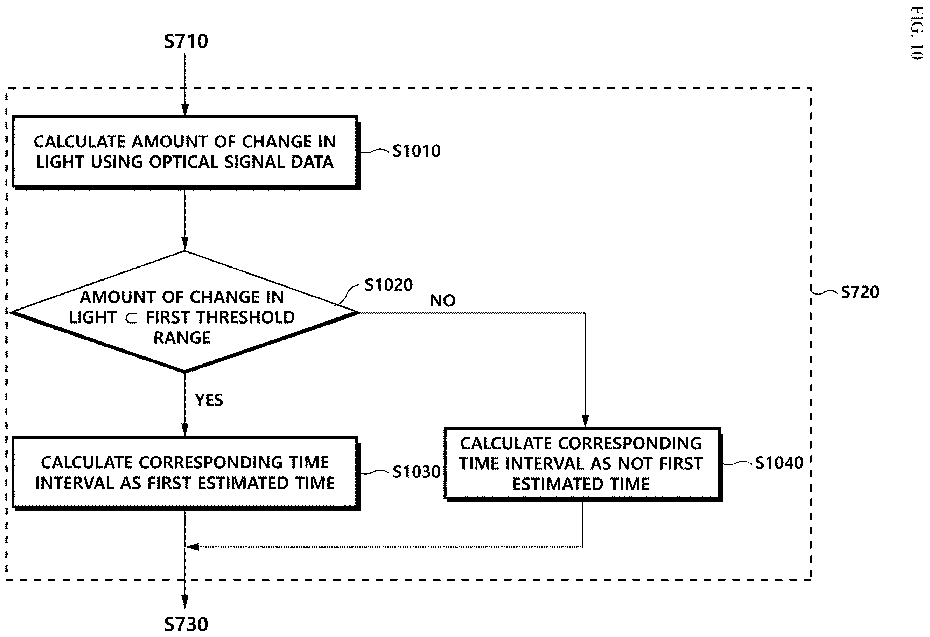

FIG. 10 is a flowchart illustrating a second embodiment of step S720 of FIG. 7.

FIG. 10 is a flowchart for detailed description in a case where the measurement data is optical signal data. With reference to FIG. 10, the step S720 of FIG. 7 may include steps S1010 to S 1040.

First, the first estimated time calculating unit 32 may calculate a amount of change in light by using information about an amount of light included in the optical signal data (S1010).

Next, the first estimated time calculating unit 32 may compare the amount of change in light with a first threshold range (S1020).

Then, the first estimated time calculating unit 32 may calculate a time interval in which the amount of change in light is included in the first threshold range, as a first estimated time (S1030).

In contrast, when the amount of change in light is not included in the first threshold range, the first estimated time calculating unit 32 may calculate the corresponding time interval as not being the first estimated time (S1040).

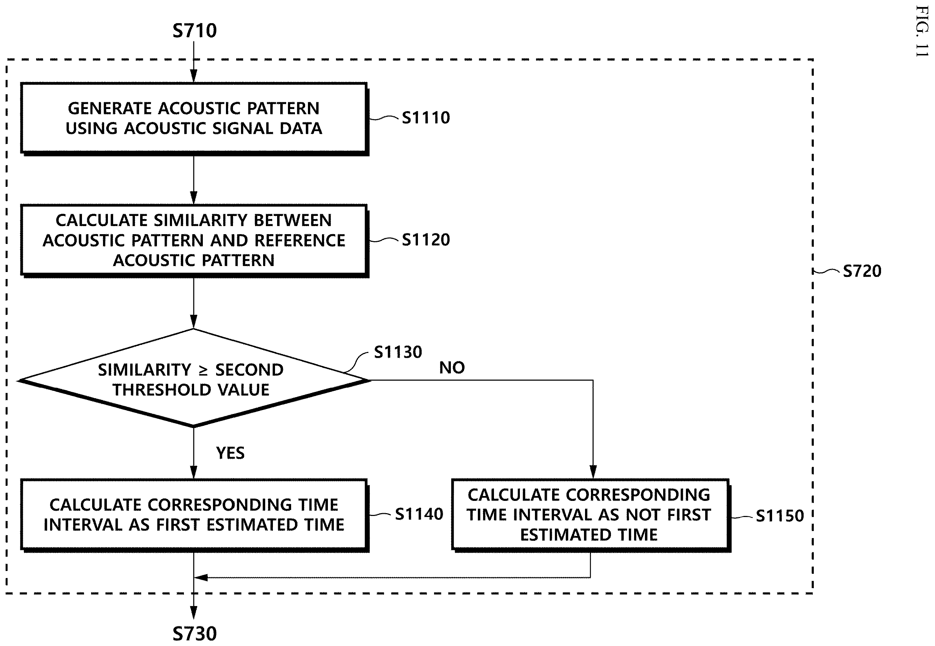

FIG. 11 is a flowchart illustrating a third embodiment of step S720 of FIG. 7.

FIG. 11 is a flowchart for detailed description in a case where the measurement data is acoustic signal data. With reference to FIG. 11, the step S720 of FIG. 7 may include steps S1110 to S1150.

First, the first estimated time calculating unit 32 may generate the acoustic pattern of acoustic signal data based on acoustic information included in the acoustic signal data (S1110).

Next, the first estimated time calculating unit 32 may calculate a similarity by comparing the acoustic pattern with a preset reference acoustic pattern (S1120).

Next, the first estimated time calculating unit 32 may determine whether the similarity is greater than or equal to a second threshold value (S1130).

Next, the first estimated time calculating unit 32 may calculate a time interval in which the similarity is greater than or equal to the second threshold value, as a first estimated time (S1140).

On the other hand, if the similarity is less than the second threshold value, the first estimated time calculating unit 32 may calculate the corresponding time interval as not being a first estimated time (S1150).

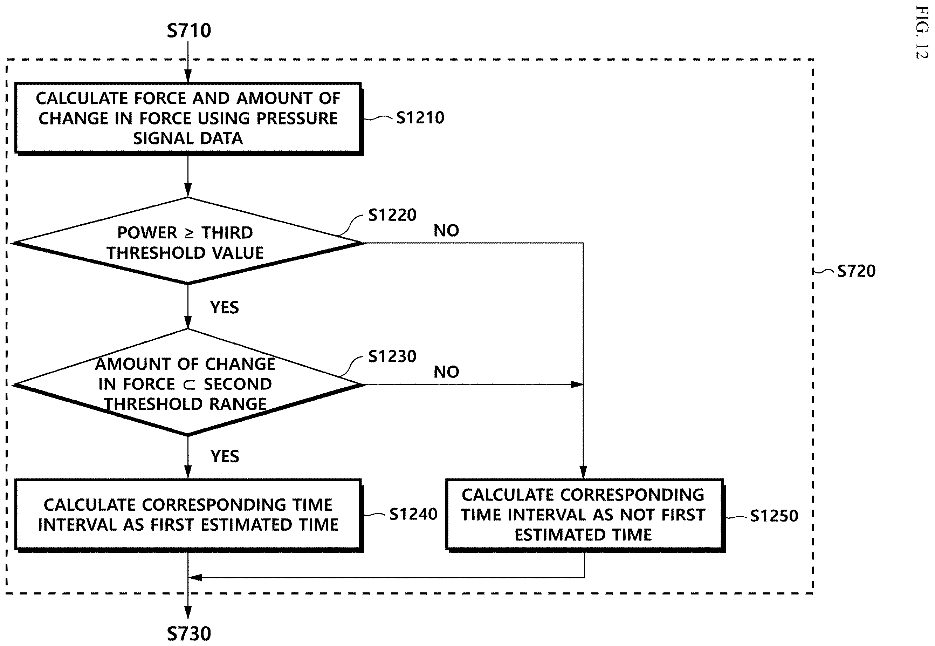

FIG. 12 is a flowchart illustrating a fourth embodiment of step S720 of FIG. 7.

FIG. 12 is a flowchart for detailed description in a case where the measurement data is pressure signal data. With reference to FIG. 12, step S720 of FIG. 7 may include steps S1210 to S1250.

First, the first estimated time calculating unit 32 may calculate force and a amount of change in force by using pressure information included in the pressure signal data (S1210).

Further, the first estimated time calculating unit 32 may determine whether a magnitude of force is greater than or equal to a third threshold value (S1220).

When the magnitude of force is greater than or equal to a third threshold value, the first estimated time calculating unit 32 may determine whether the amount of change in force is included in a second threshold range (S 1230).

When the magnitude of force is greater than or equal to the third threshold value and the amount of change in force is included in the second threshold range, the first estimated time calculating unit 32 may calculate the corresponding time interval as a first estimated time (S1240).

On the other hand, if the magnitude of force is less than the third threshold value, or the similarity is less than the predetermined threshold value, or even if the magnitude of force is greater than or equal to the third threshold value but the amount of change in force is not included in the second threshold range, the first estimated time calculating unit 32 may calculate the corresponding time interval as not being a first estimated time (S1250).

For convenience of description, the S1230 step is described as being performed after the S1220 step, but is not limited thereto. As another example, the S1230 step may be performed before the S1220 step, or the S1220 step and the S1230 step may be substantially performed simultaneously.

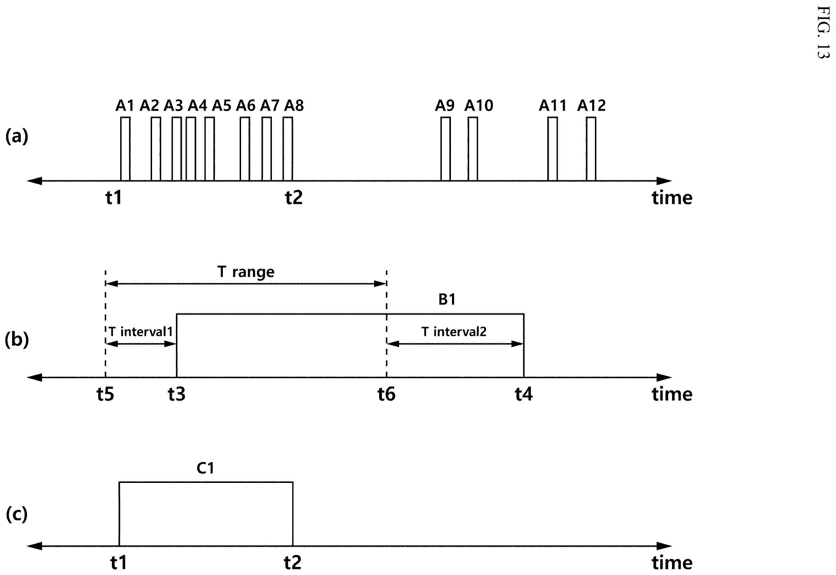

FIG. 13 is a diagram for explaining a process of calculating meal time according to an embodiment of the present invention.

According to an embodiment of the present invention, the meal time calculating unit 34 detects a meal time of a target object by using a first estimated time and a second estimated time. In this case, using the first estimated time and the second estimated time together is due to the fact that it is difficult to ensure accuracy of a calculated meal time when using only the first estimated time or only the second estimated time.

As described above, since the first estimated time uses measurement data of parameters related to an ear, a time interval related to various non-meal acts of a target object may also be calculated as a first estimated time. Accordingly, when the meal time of the target object is calculated by using only the first estimated time, a time of non-meal acts of the target object may be calculated as a meal time. That is, it may be difficult to ensure accuracy of information related to the meal time.

In addition, although the second estimated time uses blood glucose data, an increase in blood glucose cannot necessarily be determined as being related to a meal. An increase in blood glucose may occur due to various causes other than food intake, such as viral infection, lack of sleep, dehydration, smoking, or drug intake. Therefore, calculating the meal time of a target object only based on the second estimated time may also make it difficult to ensure accuracy of the calculated meal time.

Accordingly, the present invention provides high accuracy by enabling the meal time calculating unit 34 to calculate the meal time of a target object by using the first estimated time and the second estimated time together.

Meanwhile, according to one embodiment of the present invention, the first estimated time and the second estimated time may also be used to predict various blood glucose-related symptoms. For example, when a time zone estimated as a non-meal-act time through the first estimated time is determined as a second estimated time estimated as a glucose absorption time, the meal monitoring apparatus 30 may predict and provide a possibility of occurrence of diabetic ketoacidosis, in which blood glucose may sharply increase even though the target object has not eaten a meal.

Hereinafter, an example of a process of calculating a meal time of the target object by using the first estimated time and the second estimated time will be described in detail.

First, FIG. 13 (a) illustrates an example of the first estimated time calculated by the first estimated time calculating unit 32. In FIG. 13 (a), a total of twelve first estimated times (A1 to A12) are illustrated as examples.

FIG. 13 (b) illustrates an example of the second estimated time (B1) calculated by the second estimated time calculating unit 33. In FIG. 13 (b), a time period t3 to t4 is illustrated as the second estimated time.

With reference to FIG. 13 (b), a reference time range (T range) is calculated based on the second estimated time. In FIG. 13 (b), a time period t5 to t6 is illustrated as the second estimated time. According to one embodiment, as illustrated in FIG. 13 (b), a start time t5 of the reference time range (T range) may be earlier than a start time t3 of the second estimated time (B1) by a first time interval (T interval1). Further, an end time t6 of the reference time range (T range) may be earlier than an end time t4 of the second estimated time (B1) by a second time interval (T interval2). In this manner, calculating the reference time range (T range) by using the set time intervals (T interval1 and T interval2) is due to the fact that the blood glucose value does not rise immediately after food intake. Although such may vary depending on a person, a type of food, a food-intake pattern, and the like, generally, the blood glucose begins to rise about ten minutes after food intake, and reaches the highest blood glucose value after about sixty minutes. Accordingly, the present invention improves accuracy of meal-time calculation by calculating the reference time range (T range) that reflects such phenomena. Meanwhile, the time intervals (T interval1 and T interval2) used for calculating the above-described reference time range (T range) may be differently set depending on a condition of the target object. For example, for a person without diabetes, a person with type 1 diabetes, and a person with type 2 diabetes, the reference time range may be calculated by using different arbitrary times.

FIG. 13 (c) illustrates an example of the meal time calculated based on the first estimated times (A1 to A12) and the second estimated time (B1). In FIG. 13 (c), a time period t1 to t2 is illustrated as the meal time.

When comparing the first estimated times A1 to A12 illustrated in FIG. 13 (a) and the reference time range (T range) illustrated in FIG. 13 (b), first estimated times A1 to A8 are included in the reference time range (T range), whereas first estimated times A9 to A12 are not included in the reference time range (T range). Accordingly, the meal time calculating unit 34 calculates the meal time based on the first estimated times A1 to A8 among the first estimated times A1 to A12. Specifically, among the first estimated times A1 to A8, a start time t1 of the first estimated time A1, which is arranged at an earliest time, is determined as a start time of a meal. Further, among the first estimated times A1 to A8, an end time t2 of the first estimated time A8, which is arranged at the latest time, is determined as an end time of a meal.

Meanwhile, in cases of the first estimated times A9 and A10, although their time zones overlap with the second estimated time B1, they are not determined as a meal time of the target object. This is a result determined according to the reference time range calculated by using the second estimated time (B1) as non-meal acts occurring after a meal.

The term 'unit', 'part', or 'portion' used in the present embodiment means software or a hardware constituent element, such as a field programmable gate array (FPGA) or ASIC, where the term 'unit', 'part', or 'portion' performs some role. However, the term 'unit', 'part', or 'portion' is not limited to software or hardware. The term 'unit', 'part', or 'portion' may be configured to be in an addressable storage medium or configured to reproduce one or more processors. Thus, as an example, the term 'unit', 'part', or 'portion' includes constituent elements such as software constituent elements, object-oriented software constituent elements, class constituent elements, and task components, processes, functions, properties, procedures, subroutines, segments of program codes, drivers, firmware, microcode, circuitry, data, database, data structures, tables, arrays, and variables. The functions provided in the constituent elements and the term, 'units', 'parts', or 'portions' may be combined into a smaller number of constituent elements, 'units', 'parts', and 'portions' and/or divided into additional constituent elements, 'units', 'parts', and 'portions'. In addition, the constituent elements and 'units', 'parts', and 'portions' may be implemented to execute one or more CPUs within a device or secure multimedia card. While the embodiments have been described above, the embodiments are just illustrative and not intended to limit the disclosure. It can be appreciated by those skilled in the art that various modifications and applications, which are not described above, may be made to this embodiment without departing from the intrinsic features of this embodiment. For example, the respective constituent elements specifically described in the embodiments may be modified and then carried out. Further, it should be interpreted that the differences related to the modifications and alterations are included in the scope of the disclosure defined by the appended claims.

## Claims

1. A meal monitoring apparatus, comprising:
a data receiving unit configured to receive measurement data of parameters related to an ear of a target object and blood glucose data of the target object;
a first estimated time calculating unit configured to calculate a first estimated time estimated as a food intake activity time of the target object based on the measurement data;
a second estimated time calculating unit configured to calculate a second estimated time estimated as a glucose absorption time of the target object based on the blood glucose data; and
a meal time calculating unit configured to calculate a meal time of the target object based on the first estimated time and the second estimated time.

2. The meal monitoring apparatus of claim 1, wherein the first estimated time calculating unit:
compares a feature value calculated from the measurement data with a first reference value corresponding to the feature value; and
calculates the first estimated time based on a comparison result between the feature value and the first reference value.

3. The meal monitoring apparatus of claim 1, wherein the second estimated time calculating unit:
compares a blood glucose value included in the blood glucose data with a second reference value corresponding to the blood glucose value; and
calculates the second estimated time based on a comparison result between the blood glucose value and the second reference value.

4. The meal monitoring apparatus of claim 1, wherein the meal time calculating unit:
calculates a reference time range based on the second estimated time;
selects a third estimated time existing within the reference time range among the first estimated times;
detects a start estimated time being an earliest time and an end estimated time being a latest time among the third estimated times; and
calculates the meal time based on the start estimated time and the end estimated time.

5. The meal monitoring apparatus of claim 1, further comprising:
a meal amount calculating unit configured to calculate a meal amount ingested by the target object at the meal time based on the measurement data and the blood glucose data.

6. The meal monitoring apparatus of claim 1, wherein the measurement data of parameters related to the ear comprises at least one of vibration signal data, optical signal data, acoustic signal data, and pressure signal data.

7. The meal monitoring apparatus of claim 6, wherein the first estimated time calculating unit:
generates a harmonic pattern of the vibration signal data based on frequency information included in the vibration signal data;
determines whether similarity between the harmonic pattern and a preset reference harmonic pattern is greater than or equal to a first threshold value;
determines whether the frequency information is included in a predetermined frequency range; and
calculates a time interval in which the similarity is greater than or equal to a predetermined threshold value and the frequency information is included in the predetermined range as the first estimated time.

8. The meal monitoring apparatus of claim 6, wherein the first estimated time calculating unit:
calculates a amount of change in light by using information about an amount of light included in the optical signal data;
compares the amount of change in light with a first threshold range; and
calculates a time interval in which the amount of change in light is included in the first threshold range as the first estimated time.

9. The meal monitoring apparatus of claim 6, wherein the first estimated time calculating unit:
generates an acoustic pattern of the acoustic signal data based on acoustic information included in the acoustic signal data;
calculates similarity by comparing the acoustic pattern with a preset reference acoustic pattern; and
calculates a time interval in which the similarity is greater than or equal to a second threshold value as the first estimated time.

10. The meal monitoring apparatus of claim 6, wherein the first estimated time calculating unit:
calculates force and a amount of change in force by using pressure information included in the pressure signal data;
determines whether a magnitude of force is greater than or equal to a third threshold value;
determines whether the amount of change in force is included in a second threshold range; and
calculates a time interval in which the magnitude of force is greater than or equal to the third threshold value and the amount of change in force is included in the second threshold range as the first estimated time.

11. A meal monitoring method, comprising:
receiving measurement data of parameters related to an ear of a target object and blood glucose data of the target object;
calculating a first estimated time estimated as a food intake activity time of the target object based on the measurement data;
calculating a second estimated time estimated as a glucose absorption time of the target object based on the blood glucose data; and
calculating a meal time of the target object based on the first estimated time and the second estimated time.

12. The meal monitoring method of claim 11, wherein the calculating of the first estimated time comprises:
comparing a feature value calculated from the measurement data with a first reference value corresponding to the feature value; and
calculating the first estimated time based on a comparison result between the feature value and the first reference value.

13. The meal monitoring method of claim 11, wherein the calculating of the second estimated time comprises:
comparing a blood glucose value included in the blood glucose data with a second reference value corresponding to the blood glucose value; and
calculating the second estimated time based on a comparison result between the blood glucose value and the second reference value.

14. The meal monitoring method of claim 11, wherein the calculating of the meal time of the target object comprises:
calculating a reference time range based on the second estimated time;
selecting a third estimated time existing within the reference time range among the first estimated times;
detecting a start estimated time being an earliest time and an end estimated time being a latest time among the third estimated times; and
calculating the meal time based on the start estimated time and the end estimated time.

15. The meal monitoring method of claim 11, further comprising:
calculating a meal amount ingested by the target object at the meal time based on the measurement data and the blood glucose data.

16. The meal monitoring method of claim 11, wherein the measurement data of parameters related to the ear comprises at least one of vibration signal data, optical signal data, acoustic signal data, and pressure signal data.

17. The meal monitoring method of claim 16, wherein the calculating of the first estimated time comprises:
generating a harmonic pattern of the vibration signal data based on frequency information included in the vibration signal data;
determining whether similarity between the harmonic pattern and a preset reference harmonic pattern is greater than or equal to a first threshold value;
determining whether the frequency information is included in a predetermined frequency range; and
calculating a time interval in which the similarity is greater than or equal to a predetermined threshold value and the frequency information is included in the predetermined range as the first estimated time.

18. The meal monitoring method of claim 16, wherein the calculating of the first estimated time comprises:
calculating a amount of change in light by using information about an amount of light included in the optical signal data;
comparing the amount of change in light with a first threshold range; and
calculating a time interval in which the amount of change in light is included in the first threshold range as the first estimated time.

19. The meal monitoring method of claim 16, wherein the calculating of the first estimated time comprises:
generating an acoustic pattern of the acoustic signal data based on acoustic information included in the acoustic signal data;
calculating similarity by comparing the acoustic pattern with a preset reference acoustic pattern; and
calculating a time interval in which the similarity is greater than or equal to a second threshold value as the first estimated time.

20. The meal monitoring method of claim 16, wherein the calculating of the first estimated time comprises:
calculating force and a amount of change in force by using pressure information included in the pressure signal data;
determining whether a magnitude of force is greater than or equal to a third threshold value;
determining whether the amount of change in force is included in a second threshold range; and
calculating a time interval in which the magnitude of force is greater than or equal to the third threshold value and the amount of change in force is included in the second threshold range as the first estimated time.
